# EUROPEAN PATENT APPLICATION

(11) **EP 4 137 125 A1**
(43) Date of publication of application: **22.02.2023**
(21) Application number: 22460021.3
(22) Date of filing: 04.04.2022
(51) Int. Cl.: A61K 9/51

(54) **A METHOD OF OBTAINING OF LIPID NANOPARTICLES SYNTHESISED ON THE BASIS OF MARINE MICROALGAE (SCHIZOCHYTRIUM) AND LIPIDS OBTAINED FROM DIATOMS (HALAMPHORA)**

(30) Priority: 19.08.2021 PL 43876721
(71) Applicant: Uniwersytet Im. Adama Mickiewicza W Poznaniu, 61-712 Poznan (PL)
(72) Inventor: Witkowski, Andrzej, 71-043 Szczecin (PL); Dabek, Przemyslaw, 71-211 Szczecin (PL); Nowak, Izabela, 62-020 Swarzedz (PL); Marzec, Marta, 64-000 Koscian (PL)
(74) Representative: Urbanska-Luczak, Barbara

(57) **Abstract**

The subject of the invention is a method of obtaining lipid nanoparticles, synthesised on the basis of oil from marine microalgae (*Schizochytrium*) and lipids obtained from diatoms (*Halamphora*), characterised by a particle size in the range of 100-300 nm.

A manner of obtaining lipid nanoparticles synthesised on the basis of marine microalgae oil (*Schizochytrium*) and lipids obtained from diatoms (*Halamphora*) using a HSH method, consisting of a mixture containing glycerol stearate (Imwitor 900K) in an amount of 1-5 g, advantageously 2.5 g, cetyltrimethylammonium bromide (CTAB) in an mount of 0.1-0.8 g advantageously 0.4 g, lipids extracted earlier from *Halamphora* diatoms in an amount of 0.2-1 g advantageously 0.6 g, oil from marine microalgae (*Schizochytrium*) in an amount of 0.4-1.2 g advantageously 0.8 g and glycerol in an amount of 5-15 g advantageously 10 g, to a temperature above 75°C with continuous mixing at a speed of 400-600 rpm advantageously 600 rpm until an uniform consistence is obtained, then the mixture is subjected to pre-homogenisation at a speed of 8 000-24 000 rpm advantageously 13 500 rpm for 5-20 seconds, advantageously 10 seconds, afterwards to the homogenised sample 1 ml of Tween 80 aqueous water solution heated to the temperature of 40°C at concentration of 10-60% w/w, advantageously 30% w/w is added and mixed at a speed of 400-600 rpm, advantageously 600 rpm for 0.5-2 minutes advantageously for 1 minute until a uniform consistency is obtained, then the sample is subjected to two-stage proper homogenisation: in the first stage at a speed of 13 500 - 24 000 rpm advantageously 20 500 rpm for 5-15 seconds, advantageously 10 seconds; in the second stage at a speed of 8 000-13 500 rpm advantageously 9,500 rpm for 5-15 seconds, advantageously 10 seconds, after which the obtained dispersion is added to 14-14.9 ml advantageously 14.3 ml of Tween 80 water solution at concentration of 10-60% w/w advantageously 30% w/w with continuous mixing at a speed of 350-550 rpm, advantageously 350 rpm and is mixed until the system cools down to room temperature, advantageously 25°C and until NLC type lipid nanoparticles are obtained, characterised with a particle size in the range of 100-300 nm.

## Description

The subject of the invention is a method of obtaining lipid nanoparticles, synthesised on the basis of algae lipids, that is, oil from marine microalgae *(Schizochytrium)* and lipids obtained from diatoms (*Halamphora*), characterised by a particle size in the range of 100-300 nm and used in pharmaceutical and cosmetic preparations.

Lipid nanoparticles have been used as carriers of active substances in pharmaceutical products since 1990 and in cosmetic products 2005, ensuring improvement of penetration of active ingredients to the target site. The first generation of carriers are solid lipid nanoparticles (SLN), the key defect of which is relative low capacity for encapsulated active substance and their tendency for increasing loss of active compound from the lipid matrix during the storage of the manufactured dispersion. Second generation of lipid carriers are nanostructured lipid carriers (NLC), in which liquid lipids (oils) were added to the solid lipids. Compared to classic SLN, such a solution enabled the possibility of increasing the amount of incorporated active compound and improvement of physical stability of the lipid nanoparticles dispersion and of chemical stability of the encapsulated active substance. The subject matter which includes the use of lipid nanoparticles intended for pharmaceutical and cosmetic purposes was widely described in the following works: E.B. Souto, I. Baldim, W.P. Oliveira, R. Rao, N. Yadav, F.M. Gama, S. Mahant, Expert Opinion on Drug Delivery, 2020, 17(3), 357-377; E.B. Souto, R.H. Müller, Lipid Nanoparticles (Solid Lipid Nanoparticles and Nanostructured Lipid Carriers) for Cosmetic, Dermal, and Transdermal Applications, in D. Thassu (ed.), Nanoparticulate Drug Delivery Systems, CRC Press, 2007, 213-233. Moreover, NLC synthesis based on the introduction of natural oils into a lipid matrix seems justified from the point of view of reduction of risk of adverse reactions after contact with the ski, and from the point of view of properties of oils, including anti-oxidation, and even photo-protective properties [G. Badea, I. Lǎcǎtu 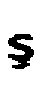 u, N. Badea, C. Ott, A. Meghea, Industrial Crops and Products, 2015, 67, 18-24], advantageous from the point of view of their later use in pharmaceutical and cosmetic products;

Selection of the method of obtaining lipid nanoparticles depends mainly on the target structure and dimension of the carrier. In case of transdermal penetration (mainly transepidermal), the 100-300 nm carrier size range predisposes them to being used as pharmaceutical/cosmetic raw materials. Many methods of obtaining lipid nanoparticles are known, including among other the method of high pressure homogenisation (HPH) using high speed homogeniser (HSH), microemulsion method or ultrasonic treatment method presented in detail in M. Uner, Pharmazie, 2006, 61, 375-386 and E.B. Souto, I. Baldim, W.P. Oliveira, R. Rao, N. Yadav, F.M. Gama, S. Mahant, Expert Opinion on Drug Delivery, 2020, 17(3), 357-377. So far, nanostructural lipid carriers containing natural oils are obtained through:
▪ High pressure homogenisation method (300-800 bar, temperature 40-70°C) with the use of, among others, buriti oil, coconut oil, Brazil nuts oil, passion fruit seed oil, andiroba oil, acai berry oil, argan oil, avocado oil, camomile oil, sunflower oil or their combination [WO2017185147A1; https://patents.google.com/patent/WO2017185147A1/en]. The synthesis proposed by the inventors is based on the use of murumuru butter in an amount of 0.5-15% w/w, a mixture of glycerol esters and polyacyladipate in an amount of 0.3-10% w/w, liquid lipid in a range of 0.01-20% w/w and surfactant in an amount 0.01-5% w/w in the lipid nanoparticles dispersion. The size of the obtained carriers fits within a range of 160-250 nm;
▪ synthesis with the use of high pressure homogenisation method (100-2000 bar, temp. 50-90 °C) with the use of *Petiolus Trachycarpi* oil and solid lipid in a 90:10 ratio [CN102283809B; https://patents.google.com/patent/CN102283809B/en]. During the synthesis the type of used hydrophilic and lipophilic surfactant was modified;
▪ Synthesis using ultrasonic treatment with the use of soybean oil or peanut oil [CN1490055A; https://patents.google.com/patent/CN1490055A/en]. The synthesis process assumes the use of fatty acid and oil, in a ratio from 10:1 to 1:1, Tween 80 as the surfactant, and preceding the ultrasonic treatment process with HSH homogenisation at a speed of 3 000-25 000 rpm. It produces NLC in the size range of 200-300 nm, stable for a period of 8 weeks.
▪ Synthesis using high pressure homogenisation (80-1000 bar) using, among others, soybean oil, sunflower oil, corn oil, olive oil, palm oil, cotton seed oil, rapeseed oil, peanut oil, coconut oil, castor oil, flaxseed oil, borage oil, evening primrose oil and additional polymer coating which protects the incorporated active substances against chemical degradation [ES2384060B1; https://patents.google.com/patent/ ES2384060B1/en]. Synthesised lipid carriers (size in the range of 102-198 nm) are used in cosmetic, pharmaceutical and food products.

The goal of the invention is the development of a method of obtaining lipid nanoparticles, synthesised on the basis of oil from marine microalgae *(Schizochytrium)* and lipids obtained from diatoms (*Halamphora*), carriers characterised by a particle size in the range of 100-300 nm, which predispose them to being used as pharmaceutical/cosmetics raw material.

The essence of the invention is a manner of obtaining lipid nanoparticles synthesised on the basis of marine microalgae oil *(Schizochytrium)* and lipids obtained from diatoms (*Halamphora*) using a HSH method, consisting of a mixture containing glycerol stearate (Imwitor 900K) in an amount of 1-5 g, advantageously 2.5 g, cetyltrimethylammonium bromide (CTAB) in an mount of 0.1-0.8 g advantageously 0.4 g, lipids extracted earlier from *Halamphora* diatoms in an amount of 0.2-1 g advantageously 0.6 g, oil from marine microalgae *(Schizochytrium)* in an amount of 0.4-1.2 g advantageously 0.8 g and glycerol in an amount of 5-15 g advantageously 10 g, to a temperature above 75°C with continuous mixing at a speed of 400-600 rpm advantageously 600 rpm until an uniform consistence is obtained, then the mixture is subjected to pre-homogenisation at a speed of 8 000 - 24 000 rpm advantageously 13 500 rpm for 5-20 seconds, advantageously 10 seconds, afterwards to the homogenised sample 1 ml of Tween 80 aqueous water solution heated to the temperature of 40°C at concentration of 10-60% w/w, advantageously 30% w/w is added and mixed at a speed of 400-600 rpm, advantageously 600 rpm for 0.5-2 minutes advantageously for 1 minute until a uniform consistency is obtained, then the sample is subjected to two-stage proper homogenisation: in the first stage at a speed of 13 500 - 24 000 rpm advantageously 20 500 rpm for 5-15 seconds, advantageously 10 seconds; in the second stage at a speed of 8 000-13 500 rpm advantageously 9 500 rpm for 5-15 seconds, advantageously 10 seconds, after which the obtained dispersion is added to 14-14.9 ml advantageously 14.3 ml of Tween 80 water solution at concentration of 10-60% w/w advantageously 30% w/w with continuous mixing at a speed of 350-550 rpm, advantageously 350 rpm and is mixed until the system cools down to room temperature, advantageously 25°C and until NLC type lipid nanoparticles are obtained, characterised by a particle size in the range of 100-300 nm.

The use of the solution presented in the invention enables the following technical and utility effects:
a. the possibility of using HSH method - that is, a method which does not require high pressure;
b. Properties of the used lipids:
   Oil from marine microalgae *(Schizochytrium)* and lipids obtained from diatoms (*Halamphora*) due to the advantageous composition of fatty acids may be successfully used for synthesis of lipid carriers as liquid lipids incorporated in an NCL lipid matrix. Oil from microalgae contains a system of polyunsaturated fatty acids - EPA eicosapentaenoic acid, DHA docosahexaenoic acid - which are responsible, among others, for protection of skin against negative effect of solar radiation, protecting it against photo-ageing and discolourations. They also aid in the treatment of eczema, ensuring a correct level of epidermal lipids [H. Bojarowicz, B. Woźniak, Problemy Higieny i Epidemiologii, 2008, 89(4), 417-475]. Similar activity is demonstrated by palmitoleic acid and palmitic acid, present in *Halamphora* diatoms, which regulate the metabolism of fatty acids within the epidermis. They foster the generation of a larger amount of lipids in lamellar bodies of the stratum granulosum of the epidermis, contributing to an increased rate of regenerative processes in the course of skin illnesses related to disorders of keratinisation [T.K. Lin, L. Zhong, J.L. Santiago, International Journal of Molecular Sciences, 2018, 19(1), 70];
c. the ability to obtain lipid carriers in the size range of 100-300 nm predisposes it to be used a pharmaceutical/cosmetic raw material;
d. the homogeneity of the particle size distribution is demonstrated by a polydispersity index (PDI) below 0.26;
e. high stability of particles is manifested by a zeta potential above +40 mV;
f. Positive charge of the obtained lipid nanoparticles is advantageous due to the presence of electrostatic attraction between negatively charged surface of the skin and the positively charged particles.

The invention is illustrated by the examples below:

### Example I

0.8 g of oil from marine microalgae *(Schizochytrium)* and 0,6 g of lipids obtained from diatoms (*Halamphora*) was combined with a mixture of molten 2.5 g of glycerol stearate, 10 g of glycerol and 0.4 g of CTAB and was mixed with a speed of of 600 rpm until a uniform consistence was obtained, and then homogenised at a speed of 13 500 rpm for 10 seconds. Afterwards, 1 ml of Tween 80 aqueous solution heated to a temperature of 40°C with a concentration of 30% w/w was added and mixed at a speed of 600 rpm for 1 minute, after which the sample was homogenised in two stages: I. at a speed of 20 500 rpm for 10 seconds; II. At a speed of 9 500 rpm for 10 seconds. A dispersion obtained in this manner was added to 14.3 ml of Tween 80 aqueous solution at concentration of 30% w/w, with continuous mixing at a speed of 350 rpm and mixed until the system cooled down to room temperature (25°C).

### Example II

Procedure was the same as in example I, changing the concentration of Tween 80 non-ionic surfactant solution to 10% w/w.

### Example III

Procedure was the same as in example I, changing the concentration of Tween 80 non-ionic surfactant solution to 60% w/w.

### Example IV

Procedure was the same as in example I, changing the amount of oil from marine microalgae *(Schizochytrium)* to 0.4 g.

### Example V

Procedure was the same as in example I, changing the amount of oil from marine microalgae *(Schizochytrium)* to 1.2 g.

### Example VI

Procedure was the same as in example I, changing the amount of glycerol stearate to 1 g.

### Example VII

Procedure was the same as in example I, changing the amount of glycerol stearate to 5 g.

### Example VIII

Procedure was the same as in example II, changing the amount of oil from marine microalgae *(Schizochytrium)* to 0.4 g.

### Example IX

Procedure was the same as in example II, changing the amount of oil from marine microalgae (*Schizochytrium*) to 1.2 g.

### Example X

Procedure was the same as in example II, changing the amount of glycerol stearate to 1 g.

### Example XI

Procedure was the same as in example II, changing the amount of glycerol stearate to 5 g.

### Example XII

Procedure was the same as in example III, changing the amount of oil from marine microalgae *(Schizochytrium)* to 0.4 g.

### Example XIII

Procedure was the same as in example III, changing the amount of oil from marine microalgae *(Schizochytrium)* to 1.2 g.

### Example XIV

Procedure was the same as in example III, changing the amount of glycerol stearate to 1 g.

### Example XV

Procedure was the same as in example III, changing the amount of glycerol stearate to 5 g.

### Example XVI

Procedure was the same as in example IV, changing the amount of glycerol stearate to 1 g.

### Example XVII

Procedure was the same as in example IV, changing the amount of glycerol stearate to 5 g.

### Example XVIII

Procedure was the same as in example V, changing the amount of glycerol stearate to 1 g.

### Example XIX

Procedure was the same as in example V, changing the amount of glycerol stearate to 5 g.

### Example XX

Procedure was the same as in example VIII, changing the amount of glycerol stearate to 1 g.

### Example XXI

Procedure was the same as in example VIII, changing the amount of glycerol stearate to 5 g.

### Example XXII

Procedure was the same as in example IX, changing the amount of glycerol stearate to 1 g.

### Example XXIII

Procedure was the same as in example IX, changing the amount of glycerol stearate to 5 g.

### Example XXIV

Procedure was the same as in example XII, changing the amount of glycerol stearate to 1 g.

### Example XXV

Procedure was the same as in example XII, changing the amount of glycerol stearate to 5 g.

### Example XXVI

Procedure was the same as in example XIII, changing the amount of glycerol stearate to 1 g.

### Example XXVII

Procedure was the same as in example XIII, changing the amount of glycerol stearate to 5 g.

### Example XXVIII

Procedure was the same as in example I, changing the amount of lipids from diatoms (*Halamphora*) to 0.2 g.

### Example XXIX

Procedure was the same as in example I, changing the amount of lipids from diatoms (*Halamphora*) to 1g.

### Example XXX

Procedure was the same as in example I, changing the amount of glycerol to 5 g.

### Example XXXI

Procedure was the same as in example I, changing the amount of glycerol to 15 g.

### Example XXXII

Procedure was the same as in example I, changing the amount of CTAB to 0.1 g.

### Example XXXIII

Procedure was the same as in example I, changing the amount of CTAB to 0.8 g.

### Example XXXIV

Procedure was the same as in example I, changing the speed of combination of the lipid phase to 400 rpm.

### Example XXXV

Procedure was the same as in example I, changing the speed of combination of the lipid phase to 500 rpm.

### Example XXXVI

Procedure was the same as in example I, changing the duration of pre-homogenisation to 5 seconds.

### Example XXXVII

Procedure was the same as in example I, changing the duration of pre-homogenisation to 20 seconds.

### Example XXXVIII

Procedure was the same as in example I, changing the speed of pre-homogenisation to 8 000 rpm.

### Example XXXIX

Procedure was the same as in example XXXVIII, changing the duration of pre-homogenisation to 5 seconds.

### Example XL

Procedure was the same as in example XXXVIII, changing the duration of pre-homogenisation to 20 seconds.

### Example XLI

Procedure was the same as in example I, changing the speed of pre-homogenisation to 24 000 rpm.

### Example XLII

Procedure was the same as in example XLI, changing the duration of pre-homogenisation to 5 seconds.

### Example XLIII

Procedure was the same as in example XLI, modifying the duration of pre-homogenisation to 20 seconds.

### Example XLIV

Procedure was the same as in example I, changing the speed of adding Tween 80 non-ionic surfactant solution to 400 rpm.

### Example XLV

Procedure was the same as in example I, modifying the speed of adding Tween 80 non-ionic surfactant solution to 500 rpm.

### Example XLVI

Procedure was the same as in example I, changing the time of mixing after adding Tween 80 non-ionic surfactant solution to 0.5 min.

### Example XLVII

Procedure was the same as in example I, changing the time of mixing after adding Tween 80 non-ionic surfactant solution to 2 min.

### Example XLVIII

Procedure was the same as in example I, modifying the speed of the 1^{st} stage of proper homogenisation to 13 500 rpm.

### Example XLIX

Procedure was the same as in example I, modifying the speed of the 1^{st} stage of proper homogenisation to 24 000 rpm.

### Example L

Procedure was the same as in example I, modifying the speed of the 2^{nd} stage of proper homogenisation to 8 000 rpm.

### Example LI

Procedure was the same as in example XLVIII, modifying the speed of the 2^{nd} stage of proper homogenisation to 8 000 rpm.

### Example LII

Procedure was the same as in example XLIX, modifying the speed of the 2^{nd} stage of proper homogenisation to 13 500 rpm.

### Example LIII

Procedure was the same as in example I, modifying the time of the 1^{st} stage of proper homogenisation to 5 seconds.

### Example LIV

Procedure was the same as in example I, modifying the time of the 1^{st} stage of proper homogenisation to 15 seconds.

### Example LV

Procedure was the same as in example I, modifying the time of the 2^{nd} stage of proper homogenisation to 5 seconds.

### Example LVI

Procedure was the same as in example I, modifying the time of the 2^{nd} stage of proper homogenisation to 15 seconds.

According to examples I- LVI nano-structural lipid carriers were obtained, characterised by a particle size (Z-Ave) in a range of **145.8-280.8 nm,** a polydispersity index (PDI) to a maximum of **0.259** and a zeta potential (ZP) of a minimum of **44.1 mV.** The detailed results are collected in the table below:

| **Example** | **Particle size (Z-Ave) [nm]** | **Polydispersity index (PDI) [-]** | **Zeta potential (ZP) [mV]** |
|---|---|---|---|
| **I.** | 194.5±3.5 | 0.194±0.014 | 50.1±0.8 |
| **II.** | 241.8±5.4 | 0.160±0.020 | 51.5±1.3 |
| **III.** | 179.9±3.2 | 0.178±0.018 | 49.910.6 |
| **IV.** | 203.6±4.2 | 0.186±0.010 | 51.4±0.7 |
| **V.** | 207.2±5.3 | 0.157±0.010 | 49.4±1.3 |
| **VI.** | 205.2±2.6 | 0.159±0.013 | 50.5±0.5 |
| **VII.** | 195.2±3.4 | 0.155±0.015 | 50.810.5 |
| **VIII.** | 196.7±6.6 | 0.175±0.006 | 48.3±1.2 |
| **IX.** | 216.6±3.1 | 0.122±0.021 | 52.7±0.6 |
| **X.** | 201.1±4.7 | 0.155±0.024 | 51.0±1.3 |
| **XI.** | 253.1±9.0 | 0.129±0.018 | 53.1±0.6 |
| **XII.** | 156.9±3.0 | 0.218±0.006 | 49.7±1.0 |
| **XIII.** | 179.9±3.1 | 0.147±0.005 | 51.2±1.9 |
| **XIV.** | 155.4±1.8 | 0.160±0.012 | 49.1±2.1 |
| **XV.** | 189.5±4.3 | 0.210±0.009 | 47.9±1.8 |
| **XVI.** | 186.8±5.3 | 0.198±0.018 | 52.6±0.2 |
| **XVII.** | 247.4±7.4 | 0.259±0.006 | 48.4±1.3 |
| **XVIII.** | 195.0±2.6 | 0.155±0.007 | 49.4±0.5 |
| **XIX.** | 215.4±4.3 | 0.178±0.005 | 48.5±1.0 |
| **XX.** | 232.615.9 | 0.166±0.016 | 53.0±1.4 |
| **XXI.** | 280.816.9 | 0.180±0.018 | 53.912.3 |
| **XXII.** | 190.1±2.2 | 0.119±0.004 | 50.6±1.0 |
| **XXIII.** | 245.7±3.3 | 0.124±0.021 | 53.1±1.4 |
| **XXIV.** | 145.8±1.6 | 0.232±0.012 | 51.1±1.0 |
| **XXV.** | 209.314.4 | 0.234±0.006 | 48.7±1.7 |
| **XXVI.** | 151.8±1.7 | 0.152±0.018 | 51.5±3.4 |
| **XXVII.** | 173.1±3.0 | 0.163±0.011 | 49.9±1.6 |
| **XXVIII.** | 175.9±0.4 | 0.158±0.006 | 45.9±1.1 |
| **XXIX.** | 230.2±4.4 | 0.195±0.008 | 52.9±1.2 |
| **XXX.** | 172.8±1.7 | 0.190±0.005 | 50.5±1.2 |
| **XXXI.** | 253.4±1.9 | 0.187±0.004 | 44.510.5 |
| **XXXII.** | 237.4±4.3 | 0.247±0.004 | 50.3±1.4 |
| **XXXIII.** | 194.4±1.3 | 0.149±0.010 | 53.212.2 |
| **XXXIV.** | 220.313.6 | 0.193±0.011 | 50.8±1.3 |
| **XXXV.** | 218.9±2.8 | 0.224±0.011 | 50.7±1.3 |
| **XXXVI**. | 211.5±6.5 | 0.250±0.010 | 49.5±0.6 |
| **XXXVII.** | 204.1±0.7 | 0.181±0.018 | 50.8±1.1 |
| **XXXVIII.** | 252.0±0.9 | 0.172±0.033 | 50.210.4 |
| **XXXIX.** | 228.9±3.1 | 0.198±0.010 | 51.4±1.2 |
| **XL.** | 248.2±3.9 | 0.224±0.018 | 51.3±1.1 |
| **XLI.** | 259.7±5.3 | 0.209±0.013 | 49.7±1.7 |
| **XLII.** | 203.0±3.7 | 0.178±0.009 | 51.7±0.6 |
| **XLIII.** | 203.9±3.6 | 0.210±0.025 | 48.9±0.3 |
| **XLIV.** | 241.4±4.1 | 0.150±0.017 | 49.9±1.6 |
| **XLV.** | 189.9±1.4 | 0.175±0.010 | 43.4±0.1 |
| **XLVI.** | 211.6±1.7 | 0.195±0.023 | 46.8±0.6 |
| **XLVII.** | 272.2±4.7 | 0.158±0.016 | 49.6±1.5 |
| **XLVIII.** | 209.5±3.1 | 0.178±0.014 | 49.0±2.3 |
| **XLIX.** | 190.6±2.3 | 0.170±0.031 | 51.4±1.3 |
| **L.** | 225.1±5.6 | 0.135±0.005 | 50.2±0.7 |
| **LI.** | 249.5±2.7 | 0.157±0.012 | 51.3±0.8 |
| **LII.** | 202.0±1.9 | 0.153±0.016 | 50.3±0.8 |
| **LIII.** | 174.2±5.2 | 0.183±0.018 | 47.3±2.6 |
| **LIV.** | 263.7±5.8 | 0.137±0.041 | 51.7±0.5 |
| **LV.** | 225.0±2.1 | 0.149±0.024 | 47.8±3.0 |
| **LVI.** | 194.2±3.4 | 0.144±0.010 | 44.1±1.2 |

Nanostructural lipid carriers obtained using the method according to the invention (example I with the use of the following amounts of substrates: 0.8 g of oil from marine microalgae *(Schizochytrium)* and 0,6 g of lipids obtained from diatoms (*Halamphora*), 2.5 g of glycerol stearate, 10 g of glycerol, 0.4 g of CTAB, Tween 80 solution at a concentration of 30% w/w) are characterised by particle size (Z-Ave) at a level of **194.5** nm, a polydispersity index (PDI) of **0.194** and zeta potential (ZP) of **50.1 mV.**

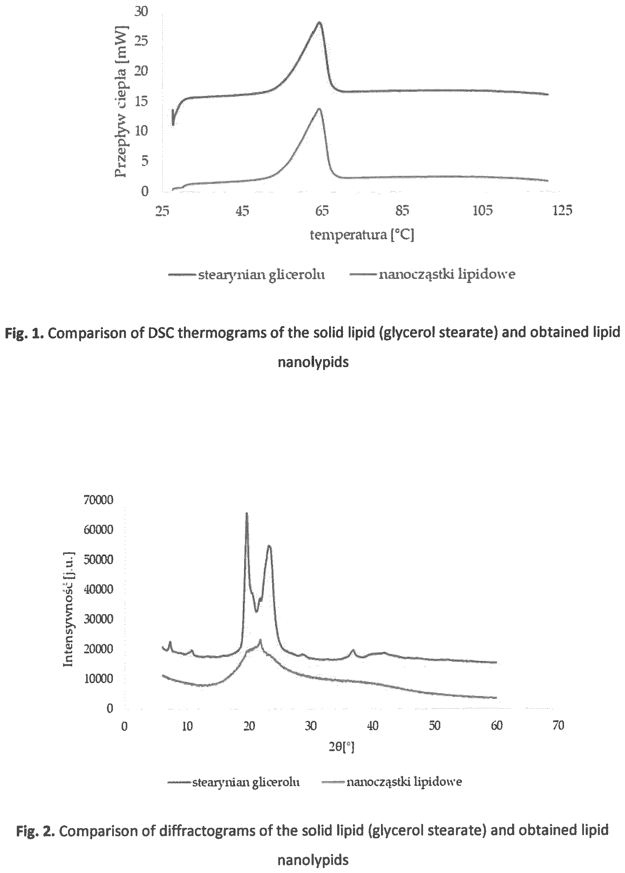

Nanostructural lipid carriers obtained using the method according to the invention were characterised by using differential scanning calorimetry (DSC) (Fig. 1) and x-ray diffraction (XRD) (Fig. 2). The conducted characteristic of the lipid matrix has confirmed the presence of the lipid in a stable polymorphic version β', characteristic for triacylglycerol based lipids in colloidal form (in dispersion of lipid nanoparticles).

### Example of use:

Lipid nanoparticles synthesised on the basis of oil from marine microalgae *(Schizochytrium)* and lipids obtained from diatoms (*Halamphora*) may be used in the cosmetic industry (carriers of active substances and their mixtures), pharmaceutical industry (for incorporation of active substances and their mixtures), food industry (to protect valuable substances, e.g. vitamins during the processing of food) and in general chemical industry (protection of select chemical compounds against degradation or succumbing to secondary and cascade reactions).

## Claims

1. manner of obtaining lipid nanoparticles synthesised on the basis of marine microalgae oil *(Schizochytrium)* and lipids obtained from diatoms (*Halamphora*) using a HSH method, **characterised in that** a mixture containing glycerol stearate (Imwitor 900K) in an amount of 1-5 g, advantageously 2.5 g, cetyltrimethylammonium bromide (CTAB) in an mount of 0.1-0.8 g advantageously 0.4 g, lipids extracted earlier from *Halamphora* diatoms in an amount of 0.2-1 g advantageously 0.6 g, oil from marine microalgae *(Schizochytrium)* in an amount of 0.4-1.2 g advantageously 0.8 g and glycerol in an amount of 5-15 g advantageously 10 g, to a temperature above 75°C with continuous mixing at a speed of 400-600 rpm advantageously 600 rpm until an uniform consistence is obtained, then the mixture is subjected to pre-homogenisation at a speed of 8 000-24 000 rpm advantageously 13 500 rpm for 5-20 seconds, advantageously 10 seconds, afterwards to the homogenised sample 1 ml of Tween 80 aqueous water solution heated to the temperature of 40°C at concentration of 10-60% w/w, advantageously 30% w/w is added and mixed at a speed of 400-600 rpm, advantageously 600 rpm for 0.5-2 minutes advantageously for 1 minute until a uniform consistency is obtained, then the sample is subjected to two-stage proper homogenisation: in the first stage at a speed of 13 500 - 24 000 rpm advantegously 20 500 rpm for 5-15 seconds, advantageously 10 seconds; in the second stage at a speed of 8 000-13 500 rpm advantageously 9 500 rpm for 5-15 seconds, advantageously 10 seconds, after which the obtained dispersion is added to 14-14.9 ml advantageously 14.3 ml of Tween 80 water solution at concentration of 10-60% w/w advantageously 30% w/w with continuous mixing at a speed of 350-550 rpm, advantageously 350 rpm and is mixed until the system cools down to room temperature, advantageously 25°C and until NLC type lipid nanoparticles are obtained, **characterised by** a particle size in the range of 100-300 nm.
